# EUROPEAN PATENT SPECIFICATION

(11) **EP 1 815 882 B1**
(45) Date of publication and mention of the grant of the patent: **30.04.2014**
(21) Application number: 04822659.1
(22) Date of filing: 22.11.2004
(51) Int. Cl.: A61N 1/36

(54) **SIMULATOR FOR PREVENTING APNEA**
SIMULATOR ZUR VERHINDERUNG VON APNOE
STIMULATEUR POUR PREVENIR L'APNEE

(43) Date of publication of application: 08.08.2007
(73) Proprietor: Techno Link Co., Ltd., Niitsu-shi, Niigata 9560804 (JP); Sasaki, Mitsuru, Iwaki-shi Fukushima 9708045 (JP)
(72) Inventor: Tsurumaki, Takeharu c/o Techno Link Co., Ltd., Niitsu-Shi Niigata; 9560804 (JP)
(74) Representative: Maillet, Alain
(86) International application number: PCT/JP2004/017337
(87) International publication number: WO 2006/054359

(56) References cited:
- WO-A1-97/49455
- WO-A2-02/13677
- JP-A- 03 023 870
- JP-A- 2001 259 048

## Description

The present invention relates to an apnea preventing stimulation apparatus for preventing the occurrence of trouble which results from abnormality in the respiratory function in one's sleep.

### BACKGROUND ART

Many studies have been reported on a so-called sleep-apnea syndrome, i.e. apnea which is accompanied by the suspension of breathing, for example, for 10 seconds or more in one's sleep at night.

If breathing comes to stop a few dozen times to hundreds of times during sleep, lack of oxygen in the body of a patient reaches a serious level, thus displaying symptoms such as insomnia and choking during sleep. As a result, the patient falls into a state of lack of sleep.

Accordingly, the patient gets sleepy in the daytime, leading to low concentration or energy level, or to dozing while working, which can cause serious accidents, such as those caused by drowsy driving.

Moreover, lack of oxygen will place undue stress on circulation organs, leading to the increased incidence of abnormal cardiac rhythm, high-blood pressure, heart failure and diabetes. Thus, the respiratory abnormality in one's sleep is a clinically important subject, and it is necessary to take measures for preventing the occurrence of the above-mentioned disorders.

The sleep-apnea syndrome is classified into a so-called central type caused by an abnormality in the respiratory center, an obstructive type by an upper airway obstruction, and a mixed type by a combination thereof.

As for the obstructive type that often causes the sleep-apnea syndrome, there have been conventionally known methods of treatment for opening the closed upper airway, by for example putting a mouthpiece into a patient's mouth to fix a lower jaw in such a manner protruding forward, or by putting on a plastic nasal cavity mask at the time of sleeping, and then pumping air from a pumping installation connected with the nasal cavity mask through a hose.

According to the former method, however, since the patient cannot take breaths through the mouth with the mouthpiece put therein, it can not be used when he/she has nasal congestion. According to the latter method, the patient must put on a nasal cavity mask for feeding air to his/her face, and thus there is a possibility that the patient may experience discomfort during sleep.

To address the foregoing problems, patent document 1 proposes an apnea preventive stimulating device in which a respiratory condition of a patient is detected by a respiration detection means such as a thermistor, and if the respiration detection means determines that the patient is in a respiratory standstill, then stimulating signals comprising electric pulses of a frequency of 40 to 150 Hz, a peak value of 1 to 50 volts and rise-up time constant of 0.2 seconds or more, are applied to his/her genioglossus muscle, which is one of the dilator muscles of the closed upper airway.

The apnea preventive stimulating device of the foregoing structure is advantageous in that since it applies stimulating signals to genioglossus muscle without using an air pressure, it is not necessary to put on a nasal cavity mask covering a substantial area on a face, and that the upper airway can be recovered from obstruction promptly, irrespective of whether the patient has nasal congestion or not.

According to the device, however, the stimulation signals are only applied when the patient is determined to be in the respiratory standstill after detecting his/her respiratory condition, two or more thermistors need to be attached to the neighborhood of both nostrils or mouth of the patient. Besides, the patient is liable to be wakened due to the stimulation signals being applied synchronously with the respiratory standstill of the patient.

A device according to the preamble of claim 1 is known from WO 021 36 77.

Patent documents 1 Japanese Registered Patent Publication No. 2794196

### DISCLOSURE OF THE INVENTION

### PROBLEMS TO BE SOLVED BY THE INVENTION

It is, therefore, an object of the present invention to provide an apnea preventing stimulation apparatus which enables stimulation signals to be effectively applied to a mental region of a patient without disturbing his/her sleep as least as possible, without the need of any respiratory condition monitoring system.

### MEANS FOR SOLVING THE PROBLEMS

The invention is defined in claim 1.

The apnea preventing stimulation apparatus according to another aspect of the invention further includes a first operation means for varying amplitude of said electric pulse.

The apnea preventing stimulation apparatus according to another aspect of the invention further includes a second operation means for varying said conduction period.

The apnea preventing stimulation apparatus according to another aspect of the invention further includes a third operation means for varying said pause period.

The apnea preventing stimulation apparatus according to another aspect of the invention further includes a fourth operation means for starting output of said stimulation signals and a fifth operation means for stopping the output thereof.

According to the apnea preventing stimulation apparatus according to a further aspect, said stimulus generating means outputs the stimulation signals in which amplitude of said electric pulse increases gradually from start of output of said stimulation signals until a first time width elapses.

The apnea preventing stimulation apparatus according to another aspect of the invention further includes a sixth operation means for varying said first time width.

According to the apnea preventing stimulation apparatus according to a still further aspect of the invention, during outputs of said electric pulse groups, said stimulus generating means outputs the stimulation signals in which a plurality of the electric pulses constituting the electric pulse groups vary in time width.

According to the apnea preventing stimulation apparatus according to a still further aspect of the invention, said stimulus generating means generates said electric pulse groups with a second time width alternately with respect to positive and negative sides during said conduction period, and outputs stimulation signals in such a way that time width of each electric pulse gradually widens from a rising edge of said electric pulse groups until half said second time width elapses and then narrows as coming closer to a falling edge of said electric pulse groups.

According to the apnea preventing stimulation apparatus according to another aspect of the invention, said stimulus generating means outputs stimulation signals in which density of a plurality of the electric pulses constituting the electric pulse groups varies during outputs of said electric pulse groups.

According to the apnea preventing stimulation apparatus according to another aspect of the invention, said stimulus generating means generates said electric pulse groups with the second time width alternately with respect to positive and negative sides during said conduction period, and outputs stimulation signals in such a way that electric pulse density increases gradually from a rising edge of said electric pulse groups until half said second time width elapses, and then decreases gradually as coming closer to a falling edge of said electric pulse groups.

According to the apnea preventing stimulation apparatus according to another aspect of the invention, said conductive unit comprises a couple of electrodes to which said stimulation signals are applied, and an adhesive sheet member that holds said electrodes and is detachably attached to a mental region of a patient.

According to the apnea preventing stimulation apparatus according to another aspect of the invention, said sheet member arranges the electrodes so that the couple of the electrodes are juxtaposed to each other on front and rear sides of a mental region of the patient.

### EFFECTS OF THE INVENTION

The stimulation signal which repeats a conduction period serving to generate an electric pulse group and a pause period to generate no electric pulse group is effectively applied from the stimulus generating means to the metal region of a patient through the conductor unit, and thus the obstruction of the upper airway is promptly removed without the need to monitor a respiratory condition of the patient.

Moreover, since the stimulation signal which repeats the conduction period and the pause period of the above-mentioned pulse group is applied to the mental region of a patient regardless of whether the patient is in a sleep apnea or not, the patient is less likely to be awakened by the stimulation and thus he/she can get sufficient sleep. Accordingly, even if the respiratory state is not monitored, the stimulation signal can be effectively applied to the mental region of the patient, with minimal disturbance in his/her sleep.

Further, the amplitude of the electric pulse generated during the conduction period can be varied by the first operation means. Accordingly, the electric pulse of optimal amplitude can be given to any patients.

Still further, the conduction period to generate the electric pulse group can be varied arbitrarily by the second operation means. Accordingly, the stimulation signal with optimal conduction period can be given to any patients.

Furthermore, the pause period to generate no electric pulse group can be varied arbitrarily by the third operation means. Accordingly, the stimulation signal with optimal pause period can be given to any patients.

Besides, by operating the fourth operation means in synchronization with bedtime hour, it is possible to output the stimulation signal to the conductor unit from that time. Moreover, when the patient is awakened for some reasons during the sleep, medical treatment can be temporarily stopped by operating the fifth operation means to stop the output of the stimulation signal. In this way, the provision of the fourth and fifth operation means enables the patient to choose whether to start or to stop outputting the stimulation signals on his/her free will.

Moreover, since the amplitude of the electric pulse is small at the time of starting the output of the stimulation signals, the influence on the sleep from the stimulation signal can be reduced to minimum. Moreover, since the amplitude of the electric pulse gradually increases as the patient falls into a sleep state, it is possible to give the patient such stimulation signal that can ensure the patient to avoid sleep apnea reliably.

Moreover, since the time taken to fall asleep varies from person to person, the variable control of the first time width by the sixth operation means will enable the stimulation signal to be given to any patients in order to allow the patient to avoid sleep apnea reliably.

Furthermore, since the stimulus generating means performs arbitrarily variable control of the time width of the individual electric pulses which constitute the electric pulse group, the waveform of the low frequency wave applied into the body of a patient can be distorted into a desirable state according to the increase or decrease of the time width of the electric pulses.

Still moreover, when the stimulus generating means outputs a stimulation signal, the electric pulse group comprising two or more high frequency signal components (electric pulses) is recurringly applied from the conductor unit to a patent as a stimulation signal, yet the waveform of each electric pulse group is distorted by the capacitive element of the patient and thus the stimulation signal takes the waveform approximated to a low frequency sine wave. Accordingly, it is possible to avoid a respiratory standstill effectively, with an extremely soft feeling of stimulation as compared with by the application of rectangular waves of the same current and frequency.

Furthermore, since the stimulus generating means performs arbitrarily variable control of the density of electric pulses which constitute each electric pulse group, the low frequency wave waveform can be distorted into a desirable state according to the density of the electric pulses in the body of a patient. Besides, the time width of each electric pulse is made constant and a pause period between the electric pulses is varied by the stimulus generating means during the output of the electric pulse groups, resulting in the absence of comparatively large-width electric pulses, which in turn means that the charge current to a patient's equivalent capacitance is supplied little by little so that the amount of charge rises gently, enabling a more physically soft feeling of stimulation to be applied.

Moreover, when the stimulus generating means outputs a stimulation signal, the waveform of each electric pulse group will be distorted in the body of a patient, and thus the stimulation signal can take such a waveform that the high frequency electric pulses are superimposed on a signal approximated to a low frequency sine wave. Accordingly, it is possible to avoid a respiratory standstill effectively, with an extremely soft feeling of stimulation as compared with by the application of rectangular wave of the same current and frequency.

Moreover, the conductor unit including the electrodes can be attached to an desired position only by sticking the sheet member to the patient's mental region, thus enabling an operator to save the trouble of attaching a pair of electrodes, one by one.

Still moreover, since a pair of electrodes can be arranged on the front and rear sides of the mental region only by sticking the sheet member on the patient's mental region, the influence of stimulation signals on the brain waves can be controlled to the minimum, enabling it to be discerned correctly whether the patient is in an asleep condition or not.

### BEST MODE FOR CARRYING OUT THE INVENTION

Next is a description of an apnea preventing stimulation apparatus in accordance with a preferred embodiment of the present invention with reference to the accompanying drawings.

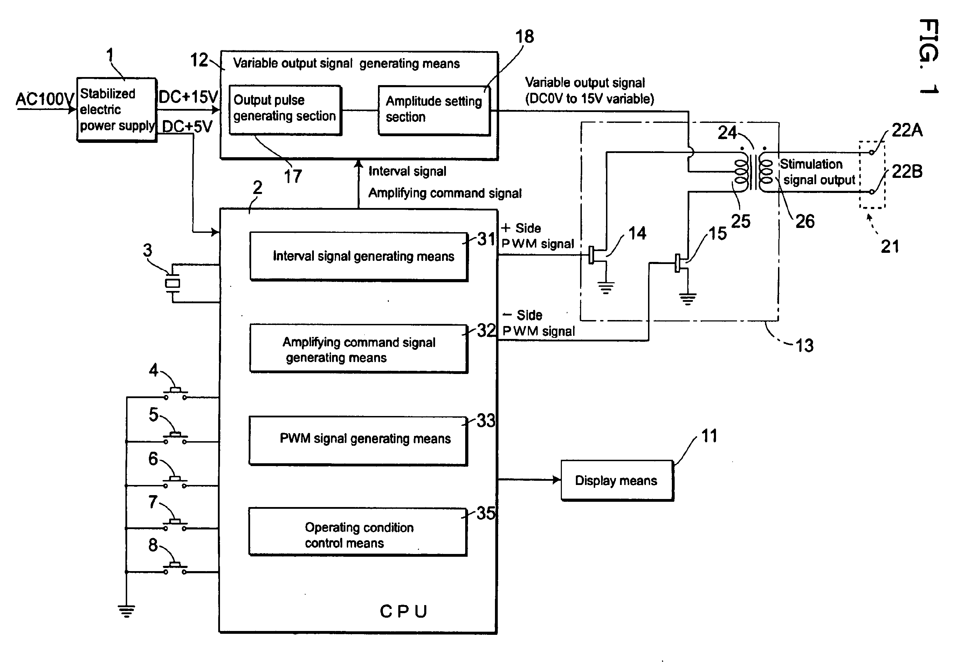

Fig. 1 is a block diagram showing an overall configuration of a apparatus in accordance with a first embodiment of the present invention. In the drawing, Fig. 1 designates a stabilized power supply for converting an AC input into a stabilized DC output. In the present embodiment, AC 100V is converted into DC+15V and DC+5V, respectively. Numeral 2 designates a CPU (central processing unit) serving as a control means actuated by the DC+5V from said stabilizing power supply 1 and reference clock signals from a crystal oscillator 3. As is well known, the CPU 2 is integrated with an input/output means, a memory means, an arithmetic processing means, etc. so that a stimulating current of a predetermined pattern may be applied to a human body as a living body (not shown) according to a control sequence memorized in the memory means.

To input ports of the CPU 2 are connected an operation means comprising a plurality of switches 4 to 8. On the other hand, to output ports thereof is connected a display means 1 comprising LEDs or LCDs, for example. Furthermore, to the output ports of the CPU 2 are connected respective gates of two FETs 14, 15 constituting a stimulus generating means 13 and a variable output signal generating means 12 for generating variable output signals to determine the amplitude, on-time and off time (pause period) of individual electric pulse groups in the stimulating signals.

Said variable output signal generating means 12 is powered by the DC voltage of DC+15V from the stabilized power supply 1, and comprises an output pulse generating section 17 which generates an output pulse in response to an interval signal as a first control signal from CPU2, and an amplitude setting section 18 which determines the amplitude of the output pulse in response to an amplifying command signal as a second control signal from CPU2,so that variable output signals of rectangular waveforms whose amplitude are modulated in a range of from DC 0V to DC+15V may be supplied to the stimulus generating means 13

The stimulus generating means 13 outputs the stimulation signals across a pair of output terminals 22A, 22B serving as the electrodes of the conductor unit 21, said stimulation signals recurring, at second-scale intervals, the conduction period in which the electric pulse groups are continuously or intermittently generated and the pause period in which no electric pulse groups are generated, by means of the variable output signals outputted from the variable output signal generating means 12 and the electric pulse generating signals (i.e., PWM signals) outputted from CPU 2 to each of FETs 14 and 15.

More specifically, the stimulus generating means 13 comprises a transformer 24 of which the primary and secondary sides are isolated from each other in addition to the said FETs 9 and 10 serving as a switching means, and the primary winding 25 of the transformer 24 has a center tap connected to a variable output signal line of said variable output signal generating means 12, while a pair of the output terminals 22A and 22B are connected to both ends of the secondary winding 26 that outputs the stimulation signals, respectively.

Moreover, one end of the primary winding 25 of the transformer 24 is connected to a drain of the source-grounded FET 14, while the other end of the primary winding 25 of the transformer 24 is connected to a drain of the source-grounded FET 15. The +side PWM signals from the CPU 2 are supplied to a gate that is a control terminal of the FET 14, while the-side PWM signals from the CPU 2 are supplied to a gate that is a control terminal of the FET 15.

The CPU 2 comprises, as a functional structure of a control sequence provided in a storing means, an interval signal generating means 31 to generate an interval signal that determines ON time and OFF time of the variable output signal; an amplifying command signal generating means 32 to generate an amplifying command signal that determines the amplitude of the variable output signal; a PWM signal generating means 33 to output a PWM signal to either one of the gates of the FETs 14 and 15; and an operating-conditions control means 35 to store operating conditions of the variable output signal and to display the operating conditions on a display means 11 or update them according to need.

The operating conditions mentioned here include: a first time width defined from the start of output of the variable output signals (eventually the stimulation signals) until the gradually increasing amplitude thereof is finally stabilized; and an amplitude of the variable output signal after the first time width, in addition to the conduction period in which the on-pulses of the variable output signals are recurringly generated and the pause period in which no on-pulses are generated.

In the present case, interval signals of the on-pulses are generated from the variable output signal means 12, and rectangular pulse groups comprising a plurality of rectangular wave pulses are outputted, as PWM signals, to the FETs 14 and 15 from PWM signal generating means 33, in synchronization with interval signals of the on-pulses being given by the interval signal generating means 31.

Preferably, the rectangular pulse groups at this time are alternately outputted to either one of the FETs 14,15 every time the interval signal of the on-pulse is generated from the variable output signal means 12, while the PWM signal generating means 33 generates PWM signals such that in each rectangular pulse group, a time width of each rectangular pulse gradually increases from the rising edge of each pulse group until the lapse of the first half of the said conduction period and then gradually decreases as it comes closer to the falling edge of the rectangular wave pulse group.

The interval signal generating means 31 is structured such that it recurringly generates the said interval signal of the on-pulse during the conduction period of the variable output signal preset and stored in said operating-conditions control means 35, but does not generate any interval signals of the on-pulse during the pause period of the variable output signal.

At the same time, the amplifying command signal generating means 32 is so structured as to generate the amplifying command signal so that the amplitude of the variable output signal may gradually increase from the start of output of the variable output signals until the lapse of the first time and then it may coincide with the preset amplitude thereof after the lapse of the first time width.

Said switch 4 is equivalent to the fourth operation means and the fifth operation means, alternately giving the CPU 2 such commands as to start or stop the output of the PWM signal, the variable output-signal, eventually the stimulation signal, every time the switch 4 is pushed. In the meantime, the fourth operation means for stating the output of the stimulation signal and the fifth operation means for stopping the output of the stimulation signal may be constructed of a separate switch, respectively.

Said switch 5 is equivalent to the first operation means for variable control of the amplitude of the electric pulse outputted as the stimulation signal such that it can vary the amplitude of the variable output signal after the lapse of the first time width preset in the operating-conditions control means 35 every time the switch 5 is pushed.

Said switch 6 is equivalent to the second operation means for variable control of the conduction period in which the electric pulse group contained in the stimulation signal is generated such that every time the switch 6 is pushed, it can change the conduction period of the variable output signal set in the operating-conditions control means 35.

Said switch 7 is equivalent to the third operation means for variable control of the pause period in which the electric pulse group contained in the stimulation signal is not generated such that whenever the switch 7 is pushed, it can vary the pause period of the variable output signal set in the operating-conditions control means 35.

Said switch 8 is equivalent to the sixth operation means for variable control of the first time width defined from the start of the output of the variable output signals or stimulation signals until the amplitude of the electric pulse group is increased and then stabilized, such that whenever the switch 8 is pushed, the first time width set in the operating-conditions control means 35 may be varied.

It should be noted that as shown in Fig. 1, the apnea preventing stimulation apparatus of the present embodiment is not provided with any respiratory-condition monitoring means for monitoring the respiratory condition of a patient. In other words, the stimulation signal generated from between the output terminals 22A, 22B of the conductor unit 21 is applied without regard to the respiratory condition of a patient.

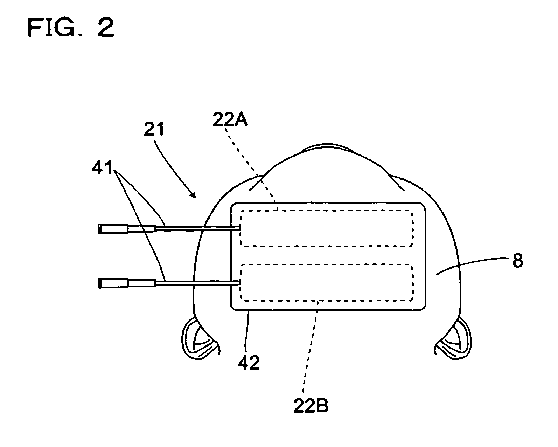

Next, the structure of the conductor unit 21 used in this apparatus is explained in more detail with reference to Fig. 2. Numerals 22A and 22B designate a pair of the output terminals mentioned above, each of which is electrically connected with the stimulus generating means 13 provided inside the main body of the apparatus (not shown) through a connecting cord 41.

Numeral 42 designates a sheet member which holds the output terminals 22A and 22B in parallel with each other. The sheet member 42 is composed of a material rich in flexibility and adhesiveness (for example, gel-like substance) so that it can be detachably attached to the mental region P of a patient. It should be particularly noted that the sheet member 42 of the present embodiment arranges the output terminals 22A and 22B in such a manner that the output terminals 22A and 22B may be arranged on the front and rear sides of the mental region P of a patient, respectively.

That is, if the electrodes of the conductor unit 21 are arranged on right and left sides of the mental region P, brain waves cannot be detected correctly when detecting the sleeping condition of a patient with brain waves, using the apparatus of the present embodiment, due to the influence of the stimulation signal applied to the electrodes. According to the present embodiment, however, the output terminals 22A and 22B are arranged on the front and rear sides of the mental region P after the sheet member 42 is stuck on the mental region P of the patient, and thus the influence of the stimulation signal on the brain waves can be reduced to the minimum, enabling it to be discerned correctly whether the patient is in an asleep condition or not.

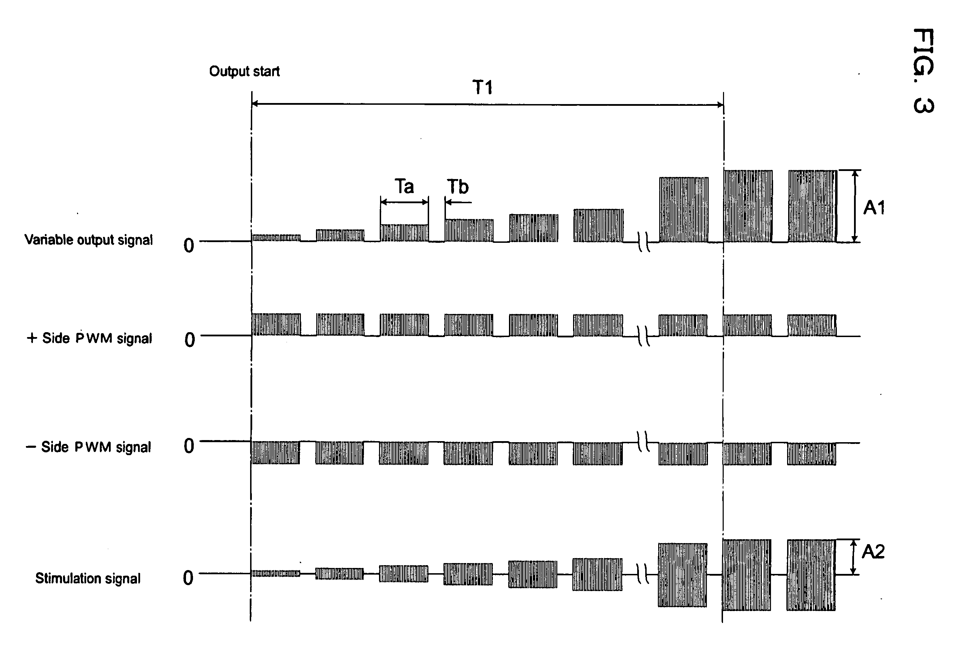

Next, the behavior of the above-configured apparatus will be described with reference to the voltage waveform diagrams shown in Figs. 3 to 5, in each of which the uppermost waveform shows a variable output signal from the output-variable circuit 11, followed by the respective waveforms of the +side PWM signals, -side PWM signals and the stimulation signals across the output electrodes 22A, 22B.

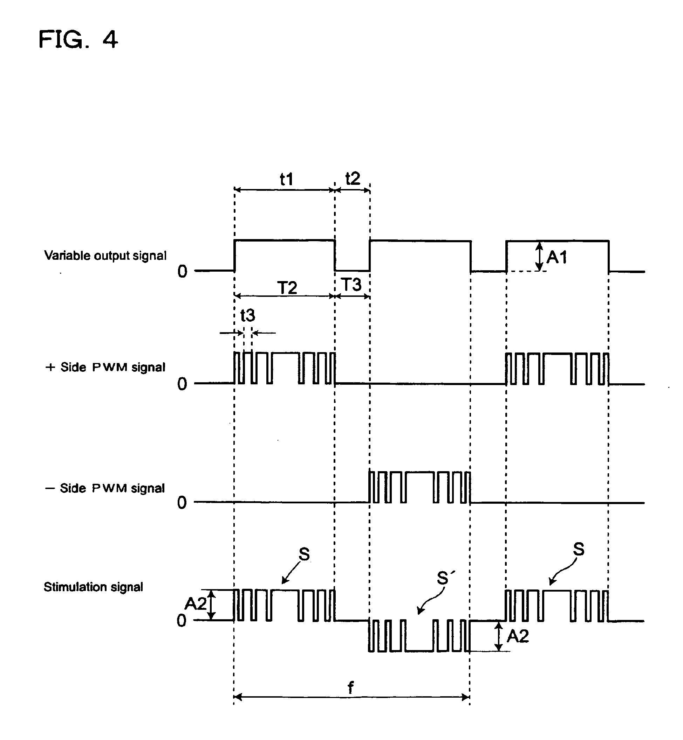

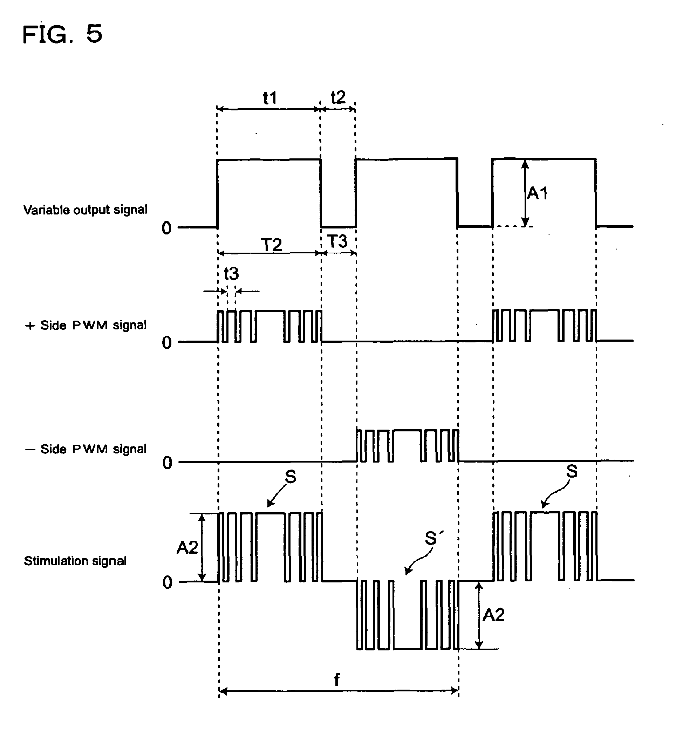

Fig. 3 is a waveform diagram of the stimulating signal showing waveforms in respective parts thereof from the start of output until lapse of the first time width. Fig.4 shows the waveforms in respective parts thereof on the way to the lapse of the first time width, while Fig.5 shows waveforms in respective parts thereof after the lapse of the first time width, respectively.

When using the apparatus of the invention, the conductor unit 2 is attached in advance to the mental region P of a patient, utilizing the adhesiveness of the sheet member 42, as shown in Fig. 2. Since the sheet member 42 is formed in an oblong shape in order for an operator to be given an proper orientation in attaching the conductor unit 21, the longer side of the sheet member 42 is inevitably attachable to the horizontal direction of the mental region P, resulting in the output terminals 22A and 22B being arranged next to each other on the front and rear sides of the mental region P.

Next, when the switch 4 is pushed to direct the start of output of the stimulation signal, the variable output signals of a plurality of on-pulses are generated at predetermined on-time t1 and off-time t2 (see Fig. 4) during the conduction period Ta shown in Fig. 3, and then during the subsequent pause period Tb, the interval signals generating no variable output signals of on-pulses are recurringly given to the output pulse generating section 17 of the variable output signal generating means 12 from the interval signal generating means 31.

At the same time, the amplifying command signal for gradually increasing the amplitude A1 of the variable output signal until the lapse of the first time width T1 (see Fig. 3) stored in the operating-conditions control means 35 is given to the amplitude setting section 18 of the variable output signal generating means 12 from the interval signal generating means 31.

As a result, during the first time width T1 from the start of output of the stimulation signal until the lapse thereof, the variable output signal with the preset ON time t1 and OFF time t2 with the amplitude A1 thereof being increased gradually, is outputted to the center tap of the primary winding 25 of the transformer 24 from the variable output signal generating means 18 during the conduction period Ta, while in the pause period Tb, such variable output signal is no longer applied to the center tap of the primary winding 25 of the transformer 24.

On the other hand, during the ON-time t1 in which the variable output signal in the form of the on pulse is output from the variable output signal generating means 12, the PWM signal generating means 33 outputs rectangular pulse group comprising a plurality of the rectangular pulses alternately to either of the gates of the FETs 14, 15 as the PWM signal.

At this time, each of the rectangular pulses has higher-frequency components than the on pulse of the variable output signal. However, until half the time width T2 (=ON time t1) in which the rectangular pulse group is output has elapsed from the rising edge of the rectangular pulse group, each rectangular pulse time width t3 gradually widens and subsequently as coming closer to the falling edge of the rectangular pulse group, each rectangular pulse time width t3 gradually narrows.

Then, when the rectangular pulse group is supplied from the PWM signal generating means 33 of the CPU 2 to the FET 14 as the +PWM signal with the variable output signal in the form of the on pulse being output to the center tap of the primary winding 25 of the transformer 24, the FET 14 is turned on while each rectangular pulse is being output, so that one end (a dotted side) of the primary winding 25 gets earthed to induce a voltage at one end (a dotted side) of a secondary winding 26. Further, similarly when the rectangular pulse group is supplied from the PWM signal generating means 33 of the CPU 2 to the FET 15 as the - PWM signal with the variable output signal in the form of the on pulse being output to the center tap of the primary winding 25 of the transformer 24, the FET 15 is turned on while each rectangular pulses is being output, so that the other end (an undotted side) of the primary winding 25 gets earthed to induce a voltage at the other end (an undotted side) of a secondary winding 26. Consequently, as shown in FIGs. 3, 4, during the conduction period Ta, every time the variable output signal in the form of the on pulse is output from the variable output signal generating means 12, the stimulation signals in which a positive electric pulse group S comprising a plurality of the electric pulses and a negative electric pulse group S' comprising a plurality of the electric pulses are alternately generated with an off period T3 intervened therebetween are applied iteratively across the output terminals 22A, 22B.

Furthermore, the amplitude A2 of each of electric pulse groups S, S' is proportional to the amplitude A1 of the variable output signal. Therefore, immediately after the output start of the stimulation signals, i.e., shortly after going to bed, the amplitude A2 of the electric pulse groups S, S' constituting the stimulation signals is so small as to be hardly perceptible, while as time goes by for the patient to fall asleep, the amplitude A2 of each of electric pulse groups S, S' increases to a level suitable for the therapy.

In the meantime, since times required for patients to fall asleep vary greatly from individual to individual, it is preferable that the time width T1 required for the electric pulse groups S, S' to increase to the level suitable for the therapy from the output start of the stimulation signals can be arbitrarily varied by an external operation. In the present embodiment, the time width T1 can be varied ranging from 0 to 30 minutes by pushing the switch 8 that is the sixth operation means.

As another modified example, without fixing a rate of increase in the amplitude A2 of each of the electric pulse groups S, S' during the time width T1, the rate of the increase may be increased over time. Thus, for a short period from the output start of the stimulation signals, the amplitude A2 of each of the electric pulse groups S, S' is increased moderately, so that the patient is less disturbed by the stimulation signals to be able to fall asleep.

According to the present embodiment, the amplitude A2 of each electric pulse within one of electric pulse groups S, S' is constant and increases gradually as time elapses with each of the electric pulse groups S, S' defined as a basic unit. The amplitude A2 of each of the electric pulses, however, may be increased gradually with each electric pulse defined as the basic unit.

When a predetermined time width T1 has elapsed, the amplitude A1 of the variable output signal approaches the amplitude of the variable output signal that is stored in the operating conditions control means 35 and is subsequent to the lapse of the first time width, and then the amplitude A2 of the electric pulse constituting the stimulation signals becomes also stable and reaches a nearly constant value. During this period of time, the stimulation signals that can rapidly prevent upper-airway obstruction is applied constantly from the output terminals 22A, 22B to the mental region P of the patient irrespective of the respiratory condition of the patient. Consequently, no means for monitoring the respiratory condition is required as has conventionally been needed and no stimulation signal occurs abruptly in synchronization with occurrence of the sleep apnea. Hence, the patient can enjoy feeling of sound sleep.

Also, after the predetermined time width T1 has elapsed, every time the variable output signal in the form of the on pulse is output from the variable output signal generating means 12 during the conduction period Ta, the PWM signal generating means 33 of the CPU 2 outputs the rectangular pulse group comprising a plurality of the rectangular pulses alternately to either of the gates of the FETs 14, 15 as the PWM signal. Thus, every time the variable output signal in the form of the on pulse is output from the variable output signal generating means 12, the positive electric pulse group S comprising a plurality of the electric pulses and the negative electric pulse group S' comprising the plurality of the electric pulses are alternately generated across the output terminals 22A, 22B with the off period T3 intervened therebetween to be applied to the mental region P of the patient as the stimulation signals.

Moreover, during the time elapsing from the rising edge of the rectangular pulse group to half the time width T2 in which the rectangular pulse group is output, the time width t3 of each rectangular pulse gradually widens, and subsequently as coming closer to the falling edge of the rectangular pulse group, the time width t3 of each rectangular pulse gradually narrows. As a result, the stimulation signals are generated across the terminals 22A, 22B in such a way that during the time elapsing from the rising edge of the electric pulse groups S, S' to half the time width T2 in which the electric pulses S, S' are output, the time width t3 of each electric pulse gradually widens, and subsequently as coming closer to the falling of the electric pulses S, S', the time width t3 of each electric pulse gradually narrows. When the stimulation signals comprising such electric pulse groups S, S' are applied to the patient (a human body), since the human body behaves like a capacitive element such as a capacitor, the higher the frequency of the signal component, the lower the impedance of the human body, so that the overall waveform of each of the electric pulse groups S, S' is distorted within the human body to form a waveform approximate to a low-frequency sinusoidal waveform. Consequently, extremely soft feeling of stimulation can be obtained as compared with a rectangular waveform with the same current and frequency. Besides, in the stimulation signals, high-frequency components remain that are obtained by switching operation of the FETs 14, 15 and therefore therapeutic effects by the high-frequency components hold promise.

Additionally, if the patient becomes awake during the therapy, then, by pushing the switch 4 to direct stoppage of outputting the stimulation signals, the variable output signal from the variable signal generating means 12 and the PWM signal from the PWM signal generating means 33 quickly stop their outputs to immediately cut off the outputs of the stimulation signals to the mental region P. As a result, the patient can get relief from uncomfortable feeling resulting from uninterrupted application of the stimulation signals at the time of awakening. Further, when getting back sleeping subsequently, only pushing the switch 4 again enables the CPU 2 to be directed to start outputting the stimulation signals and besides as no strong stimulation signal is applied directly after falling asleep, the harmful influence on sleep from the stimulation signals can be eliminated.

In a preferred example of the invention, a recurrent frequency f of the positive and negative electric pulse groups S, S' shown in FIGs. 4, 5 is 2.7 kHz, and the conduction period Ta of the variable output signal, eventually of the stimulation signal, is 30 sec., and the pause period Tb is 10 sec.. Whilst a rate of suffering the apnea varies greatly between individuals, it is preferable that the aforementioned frequency f, the conduction period Ta, and the pause period Tb may be variable arbitrarily by the external operation.

Practically, in the present embodiment, by pushing the switch 6 serving as the second operation means , the conduction period Ta of the stimulation signal in which the electric pulse groups S, S' are intermittently generated is readily variable. Further, by pushing another switch 7 serving as the third operation means , the pause period Tb of the stimulation signals in which no electric pulse groups S, S' are generated is readily variable.

Furthermore, according to the present embodiment, by pushing the switch 5 serving as the first operation means, input and output gains of the amplitude setting section 18 are changed, so that the amplitude A 1 of the variable output signal, eventually the amplitude A2 of each electric signal constituting the stimulation signals is wholly increased or decreased. Thus, if, for example, the stimulation signals are so strong as to cause awakening during sleep, the stimulation signals are adjusted by the switch 5 to decrease the amplitude A2, whereas if no sufficient therapeutic effects can be obtained to the apnea during sleep, the switch 5 can adjust the amplitude A2 to increase the amplitude A2.

Besides, though not shown, if the on period T2 and off period T3 of the electric pulse groups S, S' are made arbitrarily variable by an external operation means, more effective therapeutic effects can be obtained. In addition, this can be simply realized only by changing a control program inside the CPU 2.

According to the present embodiment as described above, there is provided the apnea preventing stimulation apparatus in which the conductive unit 21 is attached to the mental region P of the patient and the electric pulses conduct from the conductive unit 21 to the mental region P of the patient to thus apply stimuli thereto. The apnea preventing stimulation apparatus includes the stimulation generating means 13 for outputting, across the output terminals 22A, 22B, the stimulation signals that repeat alternately, for a predetermined period of time, the conduction period Ta during which the electric pulse groups S, S' comprising a plurality of the electric pulses are generated and the pause period Tb during which no electric pulse groups S, S' are generated.

In this case, the stimulation signals that alternately repeat the conduction period Ta during which the electric pulse groups S, S' are generated from the stimulation generating means 13 via the conductive unit 21 and the pause period Tb during which no electric pulse groups S, S' are generated are effectively applied to the mental region P of the patient. Consequently, even if not bothering to monitor the respiratory condition during sleep, the upper-airway obstruction can be quickly prevented. Further, since the stimulation signals that repeat alternately the conduction period Ta of the electric pulse groups S, S' and the pause period Tb thereof are applied irrespective of presence or absence of the respiration of the patient, the patient doesn't become awake by sensing the stimulation signals, resulting in sound sleep of the patient. Therefore, without mentoring the respiratory condition, the sleep of the patient becomes undisturbed as much as possible to thus enable the stimulation signals to be effectively applied to the mental region P of the patient.

Alternatively, whilst in the present embodiment, the electric pulse groups S, S' are schemed to be generated at intervals of the off period T3, the stimulation signals, however, may be schemed to generate the electric pulse groups S, S' continuously without intervening the off period T3.

Further, in the present embodiment, there is provided the switch 5 serving as the first operation means that can vary the amplitude A2 of the electric pulse contained in the stimulation signals. Thus, the amplitude A2 of the electric pulse generated during the conduction period Ta can be varied arbitrarily by the switch 5. Hence, the electric pulse of the optimal amplitude A2 can be applied to any patients.

Furthermore, according to the present embodiment, there is provided the switch 6 serving as a second operation means that can vary the conduction period Ta. Thus, the conduction period Ta during which the electric pulse groups S, S' are generated can be varied arbitrarily by the switch 6. As a result, the stimulation signals with the optimal conduction period Ta can be applied to any patients.

Moreover, in the present embodiment, there is provided the switch 7 serving as a third operation means that can vary the pause period Tb. Thus, the pause period Tb where no electric pulse groups are generated can be arbitrarily varied by the switch 7. As a result, the stimulation signals with the optimal pause period Tb can be applied to any patients.

Further, according to the present embodiment, there is provided the common switch 4 serving as the fourth operation means for starting outputting the stimulation signals and as the fifth operation means for stopping outputting the stimulation signals. In this case, when the switch 4 is operated in time with going to bed, the stimulation signals can be output to the conductive unit 21 from that time. When awakening has occurred from any cause, the therapy can be stopped temporarily by re-operating the switch 4 to stop outputting the stimulation signals. Thus, the switch 4 thus provided enables output start and output stop to be selected freely by the patient according to the patient's wishes. Additionally, the above switches 4 to 7 are not limited to a momentary type.

Furthermore, according to the present embodiment, the stimulation generating means 13 generates the stimulation signals where the amplitude A2 of the electric pulse increases gradually until the first time width T1 elapses from the output start of the stimulation signals.

Thus, the amplitude A2 of the electric pulse is small at the start of outputting the stimulation signals to enable disturbance in sleep by the stimulation signals to be constrained to the minimum. Besides, since the amplitude A2 of the electric pulse becomes larger as the patient falls asleep, the stimulation signals that can surely prevent the apnea can be applied to the patient when the patient has fallen asleep.

Moreover, according to the present embodiment, there is provided the switch 8 serving as a sixth operation means that varies the first time width T1. In this case, since the time required for getting to sleep varies between individuals, if the first time width T1 can be varied by the switch 8, the stimulation signals that can prevent surely the apnea can be applied to any patients when the patient has fallen asleep. In addition, also it goes without saying that the switch 8 is not limited to the momentary type.

Further, according to the present embodiment, there is provided the stimulation generating means 13 for outputting the stimulation signals in which a time width t3 of the plurality of the electric pulses constituting the electric pulse groups S, S' is varied during an output period of the electric pulse groups S, S'.

Thus, since the stimulation generating means 13 varies arbitrarily the time width t3 of each electric pulse constituting the electric pulse groups S, S', low-frequency waveforms entering the patient can be distorted into desirable shapes depending on variations in the time width t3 of the electric pulse.

Furthermore, according to the present embodiment, the stimulation generating means 13 generates alternately the positive and negative electric pulse groups S, S' with the second time width T2 during the conduction period Ta. Thus, the positive and negative electric pulses S, S' are alternately applied successively to the mental region P of the patient during the conduction period Ta to thereby be able to prevent the apnea reliably.

Moreover, according to the present embodiment, the stimulation generating means 13 outputs the stimulation signals in such a way that during the time elapsing from the rising edge of the electric pulses S, S' to half the second time width T2, the time width t3 of each electric pulse gradually widens and subsequently as coming closer to the falling edge of the electric pulses S, S', the time width t3 of each electric pulse gradually narrows.

Thus, the stimulation generating means 13 outputs the stimulation signals in such a way that each of electric pulse groups S, S' that comprise the plurality of the electric pulses and have the second time width T2 as a whole is generated periodically in positive and negative alternate shifts, and during the time elapsing from the rising edge of the electric pulse groups S, S' to half the second time width T2, the time width t3 of each electric pulse gradually widens and subsequently as coming closer to the falling edge of the electric pulse groups S, S', the time width t3 of each electric pulse gradually narrows. As a result, irrespective of recurrence of the electric pulse groups S, S' containing a plurality of high-frequency signal components (electric pulses) applied from the conductive unit 21 to the patient as the stimulation signals, each of the electric pulse groups S, S' is distorted by capacitive effects of the patient, so that the stimulation signals are transformed into the waveforms approximate to the low-frequency sinusoidal waveforms. Consequently, the apnea can be effectively prevented while giving extremely soft feeling of stimulation as compared to a rectangular waveform with the same current and frequency.

Further, according to the present embodiment, the conductive unit 21 comprises the output terminals 22A, 22B serving as a couple of the electrodes to which the stimulation signals are applied and the adhesive sheet member 42 that holds these output terminals 22A, 22B and is detachable in relation to the mental region P of the patient.

Thus, only sticking the adhesive sheet 42 to the mental region P of the patient enables the conductive unit 21 containing the output terminals 22A, 22B to be mounted on a desired portion. As a result, a troublesome labor for mounting each of the output terminals 22A, 22B can be saved.

Besides, the output terminals 22A, 22B in which the sheet member 42 is formed long from side to side are arranged so that the couple of the output terminals 22A, 22B are juxtaposed in the cross direction of the mental region P of the patient. As a result, only adhering the sheet member 42 to the mental region P of the patient enables the couple of the output terminals 22A, 22B to be juxtaposed in the cross direction of the mental region P of the patient. Hence, the influence on the brain wave by the stimulation signals can be restrained to a maximum extent, so that it can be precisely checked whether the patient is asleep or not.

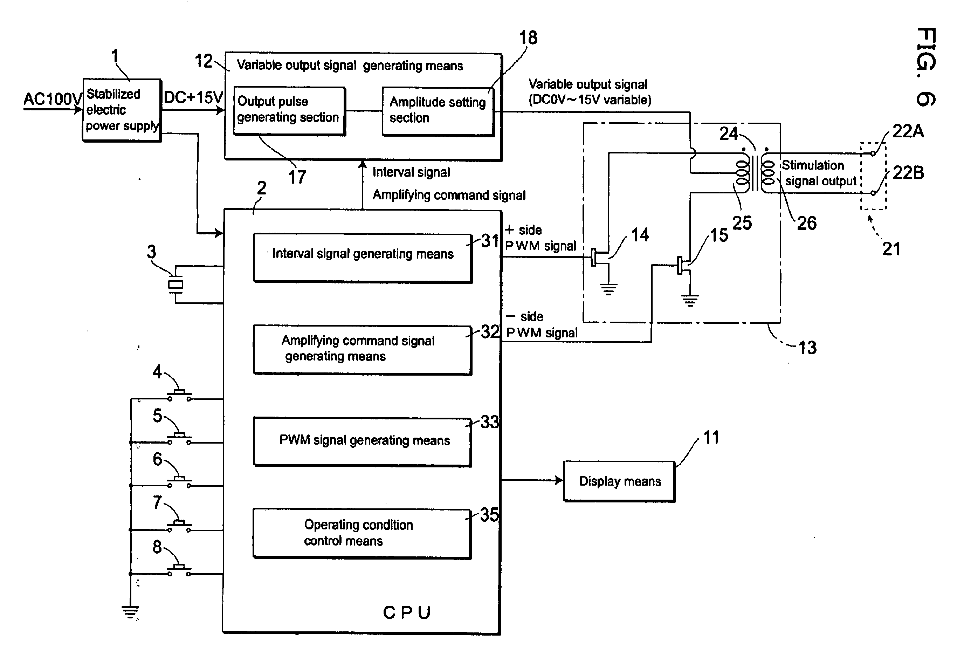

Next is a description of a second embodiment of the present invention with reference to FIG. 6 to FIG. 8. Additionally, the same numeral symbols are used for portions the same as in the first embodiment and descriptions for common portions are omitted to avoid overlap as much as possible.

In FIG. 6 showing an overall system of an apparatus, the present embodiment replaces the PWM signal generating means 33 in the first embodiment with a PDM (Pulse Density Modulation) signal generating means 52 for outputting a PDM signal to either of the gates of the FETs 14, 15. The PDM signal generating means 52 outputs rectangular pulse groups comprising a plurality of rectangular pulses to the FETs 14, 15 as the PDM signal in synchronization with interval signals in the form of the on pulse generated by an interval signal generating means 31. The rectangular pulse groups are preferably output alternately to either of the FETs 14, 15 every time the interval signal in the form of the on pulse is generated from a variable output signal means 12. Besides, it is preferable that in each of the rectangular pulse groups, each rectangular pulse is generated in such a way that time intervals (off time intervals) between adjacent rectangular pulses narrow gradually to increase pulse density per unit time during the time elapsing from the rising edge of the rectangular pulse group to half the conduction period, and subsequently as coming closer to the falling edge of the rectangular pulse group, the time intervals (the off time intervals) between adjacent rectangular pulses widen gradually to decrease the pulse density per unit time. At this case, each of the rectangular pulses has a constant time width. In addition, the remaining parts of the apparatus are in common with the first embodiment.

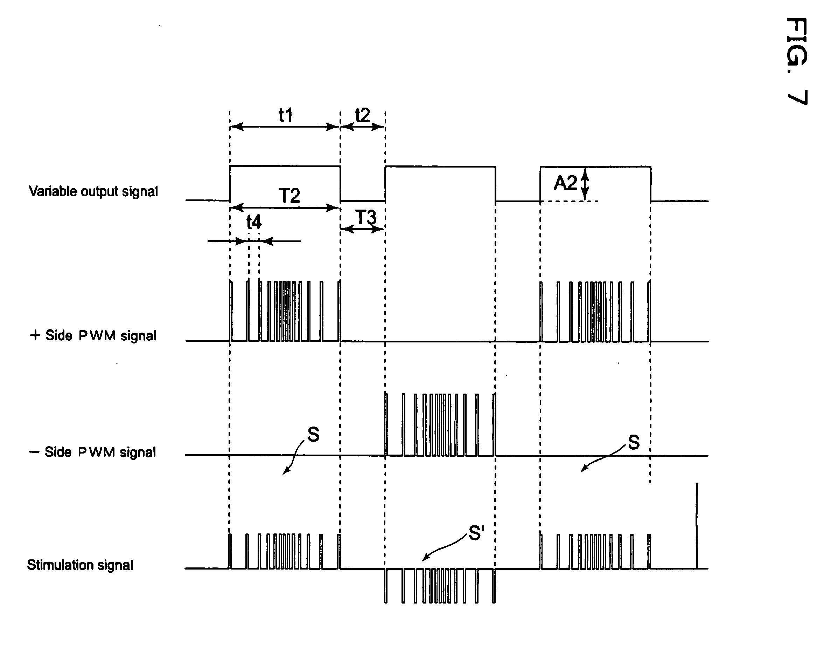

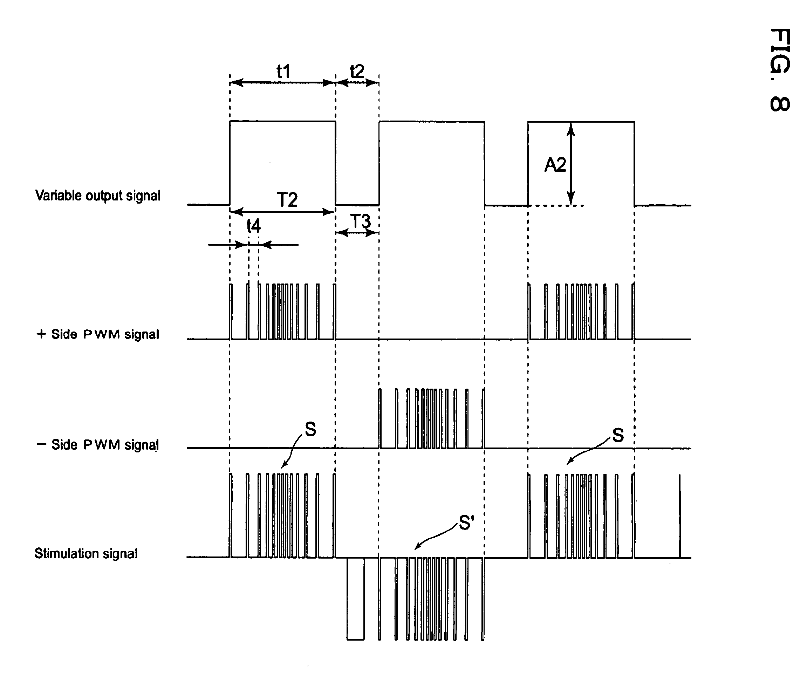

Next is a description of the performance of the aforementioned apparatus based on the waveform diagrams in FIGs. 7, 8.

In addition, FIG. 7 shows waveforms of each part in mid-course of the first time width T1, while FIG. 8 shows the waveforms of each part after the first time width T1 has elapsed.

As described in the first embodiment, when the apparatus is used, firstly, the conductive unit 21 is mounted on the mental region P of the patient using the sheet member 42 and then the stimulation signals are directed to be output by pushing the switch 4. Also in this case, immediately after the stimulation signals has started to be output, i.e., immediately after going to bed, the amplitude A2 of the electric pulse groups S, S' constituting the stimulation signals is so small as to be hardly felt by the patient. Afterward, as the time advances for the patient to get to sleep, the amplitude A2 of the electric pulse groups S, S' is increased up to a level suitable for therapy. Then, when a predetermined time width T1 has elapsed, the stimulation signals that can prevent quickly the upper-airway obstruction get applied continually to the mental region P of the patient from the output terminals 22A, 22B irrespective of the respiratory condition of the patient.

In a series of these performances, according to the present embodiment, every time the variable output signal in the form of the on pulse is output from the variable output signal generating means 12 during the conduction period Ta, the PDM signal generating means 33 of the CPU 2 outputs the rectangular pulse groups comprising a plurality of rectangular pulses alternately to either of the gates of the FETs 14, 15 as the PDM signal. Therefore, every time the variable output signal in the form of the on pulse is output from the variable output signal generating means 12, a positive electric pulse group S comprising a plurality of the electric pulses and a negative electric pulse group S' comprising a plurality of the electric pulses are generated alternately across the output terminals 22A, 22B with the off period T3 intervened therebetween, so that these pulses are applied to the mental region P of the patient as the stimulation signals.

Further, during the time elapsing from the rising edge of the rectangular pulse group to half the time width T2 where the rectangular pulse group is being generated, the off time width t4 between each of rectangular pulses narrows gradually to increase the pulse density thereof, and subsequently as coming closer to the falling edge of the rectangular pulse group, the off time width t4 between each of the rectangular pulses widens gradually to decrease the pulse density thereof. Thus, the stimulation signals are generated. Then, these stimulation signals conduct via the conductive unit 21 to the mental region P of the patient (a human body) that behaves like a capacitive element such as a capacitor.

In this case, in a portion where the off time intervals t4 between the electric pulses constituting the stimulation signals are wider, i.e., an electric pulse frequency is lower, a charge-discharge amount for an electrostatic capacity of the human body is little, so that variations in a voltage waveform between the terminals 22A, 22B becomes moderate. Adversely, in a portion where the off time intervals t4 between the electric pulses constituting the stimulation signals are narrow, i.e., the electric pulse frequency is higher, the charge-discharge amount for an equivalent electrostatic capacity of the human body is large, so that the variations in a voltage waveform between the terminals 22A, 22B becomes sharp. As a result, within the human body, the stimulation signals are modulated by a low-frequency signal approximate to a sinusoidal wave to form a waveform in which high-frequency rectangular wave signals are superimposed on the low-frequency signal. The low-frequency signal thus distorted in a sinusoidal shape can effect an extremely soft feeling of stimulation as compared to the rectangular wave with the same current and frequency. Besides, the high-frequency rectangular wave signals that are obtained by switching of the FETs 14, 15 are superimposed on the stimulation signals, so that the high-frequency components can be expected to effect a therapeutic gain.

Further, the on time width of each electric pulse is constant and the pause period (the off time interval t4) between the electric pulses varies by the stimulation generating means 13, so that no wider electric pulse generated by the PWM modulation exists. Therefore, the charging current is supplied little by little to the equivalent electrostatic capacity of the human body to raise its charged amount (an conduction amount) moderately. Accordingly, high-frequency electric pulse components can give soft feeling of stimulation.

It should be noted that in order to turn a waveform at the time of conducting to the human body into the low-frequency component approximate to the sinusoidal wave, each of the electric pulse groups S, S' that comprise a plurality of the electric pulses and have the time width T2 as a whole may be generated alternately with respect to positive and negative sides. Besides, until half the time width T2 of the electric pulse groups S, S' elapses from the rising edge of the electric pulse groups S, S', the density of each of the electric pulses becomes gradually higher, i.e., the off time intervals between each of the electric pulses become gradually narrower, and subsequently as coming closer to the falling edge of the electric pulse groups S, S', the density of each of the electric pulses becomes gradually lower, i.e., the off time intervals between each of the electric pulses become gradually wider. It is desirable that the stimulation generating means 13 be schemed to thus behave. Note, however, that if a time interval varying means for enabling the off time intervals to be varied at random is added to, e.g., the control sequence of the CPU 2 instead of the aforementioned stimulation generating means 13, not only the sinusoidal wave but a triangle wave and various distorted waves can be applied to the human body, so that a peculiar feeling of stimulation that is different from that given by the sinusoidal wave can be obtained.

According to the present embodiment as described above, during the output period of the electric pulse groups S, S', the stimulation generating means 13 is schemed so as to output the stimulation signals in which the density of a plurality of the electric pulses that constitutes the electric pulse groups S, S' is varied. In this case, since the stimulation generating means 13 varies arbitrarily the density of the plurality of the electric pulses that constitutes the electric pulse groups S, S', the low-frequency waveform entering the patient can be distorted in a desirable state depending on the variation in the density of the electric pulses. Besides, during the output period of the electric pulse groups S, S,' the time width of each electric pulse is constant and the pause period (the off time interval t4) between the electric pulses is varied by the stimulation generating means 13. Hence, by just much of absence of the electric pulse with a wider width, the charging current for the equivalent electrostatic capacity of the patient is supplied little by little to raise the charged capacity moderately, thus enabling softer feeling of stimulation to be obtained.

Further, specifically according to the present embodiment, the stimulus generating means 13 outputs the stimulation signals in such a way that during the conduction period Ta, the electric pulse groups S, S' with the second time width T2 are generated in positive and negative alternate shifts, while until half the second time width T2 elapses from the rising edge of the electric pulse groups S, S', the electric pulse density becomes gradually higher, and subsequently as coming closer to the falling edge of the electric pulse groups S, S', the electric pulse density becomes gradually lower.

When the stimulation generating means 13 outputs the stimulation signals like this, the waveform of each of the electric pulse groups S, S' is distorted within the human body, so that the stimulation signals are transformed into waveforms in which high-frequency electric pulses are superimposed on the signals approximate to the low-frequency sinusoidal waves. Consequently, the apnea can be effectively prevented while giving the extremely soft feeling of the stimulation as compared with the rectangular wave with the same current and frequency.

The present invention is not limited to each of the aforementioned embodiments and various modifications are possible within the gist of the scope of the invention. However, the stimulation generating means for outputting the desired stimulation signals to the conductive unit may be schemed by the other means than the transformer and the switching means as shown in the present embodiments, or as the control sequence of the CPU 2, the function of the variable signal generating means 12 shown in FIG. 1 may be incorporated. Further, the on time and off time of each electric pulse may be set so as to form the stimulation signals into the triangle waves and a variety of distorted waveforms by utilizing the capacitive behavior of the human body, while during the conduction period of the stimulation signals the electric pulses may be generated at random. Moreover, the electric pulse groups S, S' may comprise waveforms other than those of the PWM signal and PDM signal.

### BRIEF DESCRIPTION OF ATTACHED DRAWINGS

Fig. 1 is a block diagram showing an overall structure of an apnea preventing stimulation apparatus in accordance with a first embodiment of the invention;
Fig. 2 is an explanatory diagram showing conductor elements attached to a mental region of a patient in accordance with the first embodiment of the invention.
Fig. 3 is a waveform diagram of a stimulating signal showing waveforms in respective parts thereof from the start of output until lapse of a first time width in accordance with the first embodiment of the invention.
Fig. 4 is an enlarged waveform diagram of the stimulating signal showing waveforms in respective parts thereof on the way to the lapse of the first time width in accordance with the first embodiment of the invention.
Fig. 5 is an enlarged waveform diagram of the stimulating signal showing waveforms in respective parts thereof after the lapse of the first time width in accordance with the first embodiment of the invention.
Fig. 6 is a block diagram showing an overall structure of an apnea preventing stimulation apparatus in accordance with a second embodiment of the invention;
Fig. 7 is an enlarged waveform diagram of the stimulating signal showing waveforms in respective parts thereof on the way to the lapse of the first time width in accordance with the second embodiment of the invention.
Fig. 8 is an enlarged waveform diagram of the stimulating signal showing waveforms in respective parts thereof after the lapse of the first time width in accordance with the second embodiment of the invention.

## Claims

1. An apnea preventing stimulation apparatus for applying a stimulus to a patient, including a conductor unit to allow electric pulses to flow from the conductor unit through a mental region of the patient, comprising:
a stimulus generating means for outputting stimulation signals to said conductor unit regardless of whether the patient is in a sleep apnea or not at that moment, said stimulation signal repeats, for a given length of time, alternately a conduction period during which electric pulse groups comprising a plurality of said electric pulses are generated and a pause period during which no said electric pulse groups are generated,
**characterised in that** said stimulus generating means is adapted to output the stimulation signals such that the amplitude of said electric pulse increases gradually from start of output of said stimulation signals until a first time width elapses, and said stimulus generating means is further adapted such that
a rate of the increase in the amplitude of said electric pulses is increased over time without fixing said rate.

2. The apnea preventing stimulation apparatus according to claim 1, further comprising a first operation means for varying amplitude of said electric pulse.

3. The apnea preventing stimulation apparatus according to claim 1 or 2, further comprising a second operation means for varying said conduction period.

4. The apnea preventing stimulation apparatus according to one of claims 1 to 3, further comprising a third operation means for varying said pause period.

5. The apnea preventing stimulation apparatus according to one of claims 1 to 4, further comprising a fourth operation means for starting output of said stimulation signals and a fifth operation means for stopping the output thereof.

6. The apnea preventing stimulation apparatus according to one of claims 1 to 5, further comprising: a sixth operation means for varying said first time width.

7. The apnea preventing stimulation apparatus according to any one of claims 1 through 6, wherein during outputs of said electric pulse groups, said stimulus generating means outputs the stimulation signals in which a plurality of the electric pulses constituting the electric pulse groups vary in time width.

8. The apnea preventing stimulation apparatus according to claim 7, wherein said stimulus generating means generates said electric pulse groups with a second time width alternately with respect to positive and negative sides during said conduction period, and outputs stimulation signals in such a way that time width of each electric pulse gradually widens from a rising edge of said electric pulse groups until half said second time width elapses and then narrows as coming closer to a falling edge of said electric pulse groups.

9. The apnea preventing stimulation apparatus according to any one of claim 1 through claim 6, wherein said stimulus generating means outputs stimulation signals in which density of a plurality of the electric pulses constituting the electric pulse groups varies during outputs of said electric pulse groups.

10. The apnea preventing stimulation apparatus according to claim 8, wherein said stimulus generating means generates said electric pulse groups with the second time width alternately with respect to positive and negative sides during said conduction period, and outputs stimulation signals in such a way that electric pulse density increases gradually from a rising edge of said electric pulse groups until half said second time width elapses, and then decreases gradually as coming closer to a falling edge of said electric pulse groups.

11. The apnea preventing stimulation apparatus according to any one of claims 1 through claim 10, wherein said conductive unit comprises a couple of electrodes to which said stimulation signals are applied, and an adhesive sheet member that holds said electrodes and is detachably attached to a mental region of a patient.

12. The apnea preventing stimulation apparatus according to claim 11, wherein said sheet member arranges the electrodes so that the couple of the electrodes are juxtaposed to each other on front and rear sides of a mental region of the patient.

## Patentansprüche

1. Stimulationsvorrichtung zum Verhindern von Apnoe zum Ausüben eines Reizes auf einen Patienten, wobei die Vorrichtung eine Leitereinheit umfasst, die elektrischen Pulsen das Fließen von der Leitereinheit durch einen Mentalbereich des Patienten ermöglicht, mit:
einem Reizerzeugungsmittel zum Ausgeben von Reizsignalen an die Leitereinheit unabhängig davon, ob sich der Patient zu jenem Moment in einer Schlafapnoe befindet oder nicht, wobei das Reizsignal für eine vorbestimmte Zeitspanne alternierend eine Leiterperiode, während der elektrische Pulsgruppen, die mehrere der elektrischen Pulse umfassen, erzeugt werden, und eine Unterbrechungsperiode, während der keine dieser elektrischen Pulsgruppen erzeugt werden, wiederholt,
wobei das Reizerzeugungsmittel dazu eingerichtet ist, die Reizsignale derart auszugeben, dass die Amplitude des elektrischen Pulses von Beginn des Ausgebens des Reizsignals bis zum Ablaufen einer ersten Zeitspanne sich fortschreitend erhöht, und
wobei das Reizerzeugungsmittel ferner dazu eingerichtet ist, dass eine Rate der Erhöhung der Amplitude der elektrischen Pulse sich mit fortschreitender Zeit erhöht, ohne dass die Rate festgelegt ist.

2. Stimulationsvorrichtung zum Verhindern von Apnoe nach Anspruch 1 mit einem ersten Operationsmittel zum Variieren einer Amplitude des elektrischen Pulses.

3. Stimulationsvorrichtung zum Verhindern von Apnoe nach Anspruch 1 oder 2 mit einem zweiten Operationsmittel zum Variieren der Leiterperiode.

4. Stimulationsvorrichtung zum Verhindern von Apnoe nach einem der Ansprüche 1 bis 3 mit einem dritten Operationsmittel zum Variieren der Unterbrechungsperiode.

5. Stimulationsvorrichtung zum Verhindern von Apnoe nach einem der Ansprüche 1 bis 4 mit einem vierten Operationsmittel zum Starten einer Ausgabe der Reizsignale und mit einem fünften Operationsmittel zum Anhalten der Ausgabe der Reizsignale.

6. Stimulationsvorrichtung zum Verhindern von Apnoe nach einem der Ansprüche 1 bis 5 mit zusätzlich einem sechsten Operationsmittel zum Variieren der ersten Zeitspanne.

7. Stimulationsvorrichtung zum Verhindern von Apnoe nach einem der Ansprüche 1 bis 6, bei welcher das Reizerzeugungsmittel während des Ausgebens der elektrischen Pulsgruppen die Reizsignale ausgibt, in denen mehrere der elektrischen Pulse, welche die elektrischen Pulsgruppen bilden, in ihrer Zeitdauer variieren.

8. Stimulationsvorrichtung zum Verhindern von Apnoe nach Anspruch 7, bei welcher das Reizerzeugungsmittel die elektrischen Pulsgruppen mit einer zweiten Zeitspanne alternierend bezüglich positiver und negativer Seiten während der Leiterperiode erzeugt und Reizsignale derart ausgibt, dass sich eine Zeitdauer jedes elektrischen Pulses von einer ansteigenden Kante der elektrischen Pulsgruppen bis zum Ablauf der Hälfte der zweiten Zeitspanne fortschreitend vergrößert und dann bei Annäherung an eine fallende Kante der elektrischen Pulsgruppen abnimmt.

9. Stimulationsvorrichtung zum Verhindern von Apnoe nach einem der Ansprüche 1 bis 6, bei welcher das Reizerzeugungsmittel Reizsignale ausgibt, bei denen eine Dichte mehrerer der elektrischen Pulse, welche die elektrischen Pulsgruppen bilden, während der Ausgabe der elektrischen Pulsgruppen variiert.

10. Stimulationsvorrichtung zum Verhindern von Apnoe nach Anspruch 8, bei welcher das Reizerzeugungsmittel die elektrischen Pulsgruppen mit der zweiten Zeitspanne alternierend bezüglich der positiven und negativen Seiten während der Leiterperiode erzeugt und Reizsignale derart ausgibt, dass eine elektrische Pulsdichte von einer ansteigenden Kante der elektrischen Pulsgruppen bis zum Ablauf der Hälfte der zweiten Zeitspanne fortschreitend ansteigt und dann bei Annäherung an eine fallende Kante der elektrischen Pulsgruppen fortschreitend abnimmt.

11. Stimulationsvorrichtung zum Verhindern von Apnoe nach einem der Ansprüche 1 bis 10, bei welcher die Leitereinheit ein Elektrodenpaar, an welches die Reizsignale angelegt werden, und ein haftendes Blattelement, welches die Elektroden trägt und lösbar mit einem Mentalbereich eines Patienten verbunden ist, umfasst.

12. Stimulationsvorrichtung zum Verhindern von Apnoe nach Anspruch 11, bei welcher das Blattelement die Elektroden derart anordnet, dass das Elektrodenpaar auf der Vorderseite und der Rückseite eines Mentalbereichs des Patienten nebeneinander angeordnet ist.

## Revendications

1. Un appareil de stimulation prévenant l'apnée pour appliquer un stimulus à un patient, comprenant une unité conductrice pour permettre à des impulsions électriques de se propager de l'unité conductrice jusqu'à une région mental du patient, comportant:
un moyen de génération de stimulus pour émettre en sortie des signaux de stimulation à ladite unité conductrice indépendamment de si le patient est en apnée du sommeil ou non à cet instant, ledit signal de stimulation répète, pour une durée donnée, alternativement une période de conduction durant laquelle des groupes d'impulsions électriques comprenant une pluralité desdites impulsions électriques sont générées et une période de pause pendant laquelle aucun desdits groupes d'impulsions électriques n'est généré,
**caractérisé en ce que** ledit moyen de génération de stimulus est adapté pour émettre les signaux de stimulation de manière à ce que l'amplitude desdites impulsions électriques augmente progressivement du début de l'émission desdits signaux de stimulation jusqu'à l'écoulement d'une première largeur de temps, et ledit moyen de génération de stimulus est en outre adapté pour que un taux d'augmentation de l'amplitude desdites impulsions électriques augmente au cours du temps sans fixation dudit taux.

2. L'appareil de stimulation prévenant l'apnée selon la revendication 1, comportant en outre un premier moyen d'opération pour faire varier l'amplitude de ladite impulsion électrique.

3. L'appareil de stimulation prévenant l'apnée selon la revendication 1 ou 2, comportant en outre un second moyen d'opération pour faire varier ladite période de conduction.

4. L'appareil de stimulation prévenant l'apnée selon l'une des revendications 1 à 3, comportant en outre un troisième moyen d'opération pour faire varier ladite période de pause.

5. L'appareil de stimulation prévenant l'apnée selon l'une des revendications 1 à 4, comportant en outre un quatrième moyen d'opération pour démarrer l'émission desdits signaux de stimulation et un cinquième moyen d'opération pour arrêter l'émission de ceux-ci.

6. L'appareil de stimulation prévenant l'apnée selon l'une des revendications 1 à 5, comportant en outre un sixième moyen d'opération pour faire varier ladite première largeur de temps.

7. L'appareil de stimulation prévenant l'apnée selon l'une des revendications 1 à 6, dans lequel au cours des émissions desdits groupes d'impulsions électriques, ledit moyen de génération de stimulus émet les signaux de stimulation, parmi lesquels une pluralité des impulsions électriques constituant les groupes d'impulsions électriques varie en largeur de temps.

8. L'appareil de stimulation prévenant l'apnée selon la revendication 7, dans lequel ledit moyen de génération de stimulus génère lesdits groupes d'impulsions électriques avec une seconde largeur de temps en alternance par rapport aux côtés positifs et négatifs durant ladite période de conduction, et émet des signaux de stimulation, de telle sorte que la largeur de temps de chaque impulsion électrique s'élargit progressivement à partir d'un front montant desdits groupes d'impulsions électriques jusqu'à ce que la moitié de ladite seconde largeur de temps s'écoule et se rétrécit ensuite en se rapprochant d'un front descendant desdits groupes d'impulsions électriques.

9. L'appareil de stimulation prévenant l'apnée selon l'une des revendications 1 à 6, dans lequel ledit moyen de génération de stimulus émet des signaux de stimulation, dans lesquels la densité d'une pluralité d'impulsions électriques constituant les groupes d'impulsions électriques varie au cours de l'émission desdits groupes d'impulsions électriques.

10. L'appareil de stimulation prévenant l'apnée selon la revendication 8, dans lequel ledit moyen de génération de stimulus génère lesdits groupes d'impulsions électriques avec la seconde largeur de temps en alternance par rapport aux côtés positifs et négatifs durant ladite période de conduction, et émet des signaux de stimulation de telle sorte que la densité d'impulsion électrique augmente progressivement à partir d'un front montant desdits groupes d'impulsions électriques jusqu'à ce que la moitié de ladite seconde largeur de temps s'écoule, et ensuite diminue progressivement en se rapprochant d'un front descendant desdits groupes d'impulsions électriques.

11. L'appareil de stimulation prévenant l'apnée selon l'une des revendications 1 à 10, dans lequel ladite unité conductrice comprend un couple d'électrodes auxquelles lesdits signaux de stimulation sont appliqués, et une feuille adhésive qui maintient lesdites électrodes et qui est fixée de manière amovible à une région mentale d'un patient.

12. L'appareil de stimulation prévenant l'apnée selon la revendication 11, dans lequel ladite feuille dispose les électrodes de telle sorte que le couple d'électrodes sont juxtaposées les unes aux autres sur les côtés avant et arrière d'une région mental du patient.
